# FASCICULE DE BREVET EUROPEEN

(11) **EP 2 737 072 B1**
(45) Date de publication et mention de la délivrance du brevet: **14.02.2018**
(21) Numéro de dépôt: 12753763.7
(22) Date de dépôt: 27.07.2012
(51) Int. Cl.: C12N 15/866, C12N 7/00

(54) **SYSTEME BACULOVIRAL POUR L'EXPRESSION D'UN VECTEUR DE THERAPIE GENIQUE**
BACULOVIRUSSYSTEM ZUR EXPRESSION EINES GENTHERAPIEVEKTORS
BACULOVIRUS SYSTEM FOR THE EXPRESSION OF A GENE THERAPY VECTOR

(30) Priorité: 27.07.2011 FR 1156878
(43) Date de publication de la demande: 04.06.2014
(73) Titulaire: Genethon, 91000 Evry (FR)
(72) Inventeur: GALIBERT, Lionel, F-92300 Levallois-Perret (FR); MERTEN, Otto-Wilhelm, F-78121 Crespières (FR); JACOB, Aurélien, F-92120 Montrouge (FR)
(74) Mandataire: Sekhri, Redha
(86) Numéro de dépôt international: PCT/FR2012/051791
(87) Numéro de publication internationale: WO 2013/014400

(56) Documents cités:
- WO-A1-2004/022760
- WO-A1-2008/099148
- Gailbert, L.; Rivière, C.; Cohen, D.P.A.; Oers, M.M. van; Merten, O.W.: "Baculovirus deleted for chitinase, cathepsin and p10 genes improves purified rAAV8 quality", Advanced Symposium and EMBO Practical Course on Viral Vectors in Gene Therapy: Applications & Novel Production Methods, Kuopio, Finland, 25 August - 4 September 2010. Abstract in scientific journal or proceedings, 2010, XP002667811, Extrait de l'Internet: URL:http://library.wur.nl/WebQuery/wurpubs /396681 [extrait le 2012-01-24]
- SMITH RICHARD H ET AL: "A simplified baculovirus-AAV expression vector system coupled with one-step affinity purification yields high-titer rAAV stocks from insect cells.", MOLECULAR THERAPY : THE JOURNAL OF THE AMERICAN SOCIETY OF GENE THERAPY NOV 2009 LNKD- PUBMED:19532142, vol. 17, no. 11, novembre 2009 (2009-11), pages 1888-1896, XP002667812, ISSN: 1525-0024 cité dans la demande
- ASLANIDI GEORGE ET AL: "An inducible system for highly efficient production of recombinant adeno-associated virus (rAAV) vectors in insect Sf9 cells", PROCEEDINGS OF THE NATIONAL ACADEMY OF SCIENCES OF THE UNITED STATES OF AMERICA, vol. 106, no. 13, mars 2009 (2009-03), pages 5059-5064, XP002667813, ISSN: 0027-8424 cité dans la demande
- NOAD ROB J ET AL: "Multigene expression of protein complexes by iterative modification of genomic Bacmid DNA", BMC MOLECULAR BIOLOGY, BIOMED CENTRAL LTD, GB, vol. 10, no. 1, 2 septembre 2009 (2009-09-02), page 87, XP021056445, ISSN: 1471-2199, DOI: 10.1186/1471-2199-10-87 cité dans la demande
- WANG C: "Hybrid baculovirus-adeno-associated virus vectors for prolonged transgene expression in human neural cells", JOURNAL OF NEUROVIROLOGY, INFORMA HEALTHCARE, GB, vol. 14, no. 6, 1 novembre 2008 (2008-11-01), pages 563-568, XP009155644, ISSN: 1355-0284
- GRAY JT ET AL: "Design and construction of functional AAV vectors", 1 janvier 2011 (2011-01-01), ADENO-ASSOCIATED VIRUS,, PAGE(S) 25 - 46, XP009155664, page 31

## Description

Les virus adéno-associés recombinants (ou rAAV, pour recombinant Adeno Associated Virus en anglais) sont actuellement considérés comme les vecteurs viraux les plus prometteurs en thérapie génique. Cependant, leur production à grande échelle reste un facteur limitant pour le développement de ce type de thérapies. Des systèmes de production exploitant la capacité des baculovirus à infecter des cellules d'insecte ont donc été développés en vue de pallier ce problème, et pour des raisons de biosécurité (Urabe et al. 2002; Hum. Gene Ther. 13: 1935-1943; US 20030148506; US 20040197895). Selon le protocole initialement proposé, une infection des cellules d'insecte par 3 baculovirus différents était nécessaire pour fournir les gènes helper *rep* et *cap* et la construction comprenant le vecteur AAV recombinant contenant le transgène requis pour former la particule virale recombinante. Une simplification de ce système consiste en l'intégration des gènes *rep* et *cap* dans un baculovirus unique résultant en un système de production utilisant 2 baculovirus (bac rep/cap et bac-transgène) (Smith et al. 2009; Mol. Ther. 17: 1888-1896). Par ailleurs, le groupe de Sergei Zolotukhin (Aslanidi et al. 2009; Proc. Natl. Acad. Sci. USA 206: 5059-5064 et WO 2010/114948) a récemment développé des lignées cellulaires Sf9 stablement transformées avec les gènes *rep* et *cap.* Avec une telle lignée cellulaire, l'infection par un seul baculovirus (comprenant dans son génome le le gène thérapeutique d'intérêt flanqué d'ITR d'un AAV) permettrait l'obtention d'un AAV recombinant. Cependant, ce système présente plusieurs inconvénients. D'une part, la génération de clones cellulaires est fastidieuse et les clones obtenus sont souvent caractérisés par une instabilité génétique qui résulte en une fenêtre d'exploitation limitée dans le temps. En outre, une fréquence élevée d'empaquetage d'ADN étranger qui ne correspond pas au vecteur d'intérêt (séquences de *rep*, *cap* et de gènes de résistance à un antibiotique) a été observée, ce qui pose un problème considérable sur le plan de la biosécurité. De tels clones sont donc impropres à la production de lots de vecteurs destinés à une utilisation clinique.

Tous les baculovirus utilisés dans les études mentionnées ci-dessus comprennent l'insertion du gène d'intérêt (qu'il s'agisse des gènes *rep* et/ou *cap* et du gène thérapeutique d'intérêt) dans le site de clonage polyédrine du génome baculoviral. Ce site est classiquement choisi en raison des forts niveaux d'expression susceptibles d'être obtenus à partir de ce locus. Ce site est également exploité dans le système développé par Luckow et al. (1993 ; J. Virol. 67:4566-4579) dans lequel est inséré une origine de réplication bactérienne, un gène de résistance à la kanamycine et un site de clonage par recombinaison « Tn7 ». Ce système est appelé bacmide. Cependant, l'utilisation de ce seul locus constitue un facteur limitant, notamment si l'on souhaite pouvoir exprimer plusieurs séquence hétérologues à partir d'un baculovirus unique. C'est dans ce contexte que Noad et al. (2009) ont comparé le locus classique - polyédrine (Tn7 du bacmide, présent au site polyédrine) - à différents sites de clonage potentiels et ont identifié 7 loci additionnels (*ctx, egt, 39k, orf51, gp37, iap2, odv-e56*) dans le génome d'AcMNPV permettant une forte expression de gènes hétérologues (Noad et al. 2009; BMC Molecular Biology 10: 87; WO 2010/055292). Il a également été montré que différents gènes clonés dans plusieurs de ces loci peuvent être exprimés de manière concomitante à partir du même génome. Noad et al. n'ont toutefois pas montré si ces loci alternatifs étaient efficaces pour la production d'AAV recombinant ou tout autre vecteur viral recombinant, ce qui n'était pas évident compte tenu de la complexité de ces vecteurs viraux et des difficultés majeures rencontrées classiquement lors de leur production.

### Résumé de l'invention

De manière surprenante, nous avons pu montrer que des AAV recombinants peuvent être produits grâce à un baculovirus unique.

L'invention concerne donc un génome baculoviral recombinant comprenant des séquences hétérologues codant l'ensemble des composantes nécessaires à la production d'un vecteur viral hétérologue (c'est-à-dire les composantes protéiques du vecteur et son génome) insérés dans des locus choisis dans le groupe constitué des gènes non-essentiels du baculovirus qui peuvent être remplacés par une séquence d'intérêt sans modifier le fonctionnement du baculovirus.

L'invention concerne en particulier un génome baculoviral recombinant comprenant:
- une ou plusieurs cassettes d'expression des gènes *rep* et *cap* d'AAV nécessaires à la production d'un vecteur AAV, et
- un génome recombinant d'un vecteur AAV (également appelé génome hétérologue par la suite),
   lesdites cassettes d'expression et ledit génome recombinant d'un vecteur AAV étant insérés dans un ou plusieurs locus choisi dans le groupe constitué des locus *egt, polyédrine, ctx, 39k, orf51, gp37, iap2* et *odv-e56, p10 et p94,*
   ledit génome recombinant d'un vecteur AAV étant inséré dans un locus différent du(des) locus utilisés pour les gènes *rep* et *cap* d'AAV. Selon une variante, lesdits gènes *rep* et *cap* sont contenus dans une cassette d'expression unique, notamment en orientation inverse, ladite cassette étant plus particulièrement insérée dans le locus *egt.* Dans un mode particulier de réalisation, ledit génome recombinant d'un vecteur AAV est inséré au niveau du locus *polyédrine*.

Les génomes baculoviraux recombinant selon l'invention peuvent notamment être dérivés du baculovirus AcMNPV. Par ailleurs, le génome baculoviral recombinant de l'invention peut également avoir pour caractéristique d'être déficient pour les gènes *chitinase, cathépsine* et *p10*.

Le génome d'AAV peut comprendre un gène hétérologue codant une protéine, un ARN interférant ou un ARN antisens, en particulier thérapeutique (pour permettre la production d'un vecteur viral de thérapie génique).

Selon un mode particulier de réalisation, le génome baculoviral recombinant est un bacmide recombinant.

L'invention concerne en outre un baculovirus recombinant qui a pour génome un génome baculoviral recombinant, notamment un bacmide, selon l'invention.

L'invention concerne par ailleurs un procédé pour produire un baculovirus recombinant, comprenant la culture d'une cellule procaryote contenant le bacmide recombinant défini dans la présente demande dans des conditions adaptées à la production d'un baculovirus.

L'invention fournit également une cellule eucaryote ou procaryote contenant le génome baculoviral recombinant divulgué, ou infectée par le baculovirus recombinant de l'invention. Ladite cellule peut notamment être une cellule de mammifère (par exemple une cellule HEK293) ou une cellule d'insecte dérivée des lignées *Spodoptera frugiperda* ou *Trichoplusia ni* (par exemple les cellules Sf21, Sf9, TN 5B1-4 ou High Five).

L'invention concerne en outre un procédé pour produire un AAV recombinant comprenant la mise en culture du baculovirus recombinant comprenant les séquences nécessaires pour la production des composantes protéiques et génétiques d'un AAV, avec une cellule, notamment une cellule d'insecte (par exemple une cellule Sf9, Sf21, TN 5B1-4 ou High Five), susceptible d'être infectée par ledit baculovirus, dans des conditions permettant l'infection de la cellule par le baculovirus et la production dudit AAV recombinant.

### Légende des figures

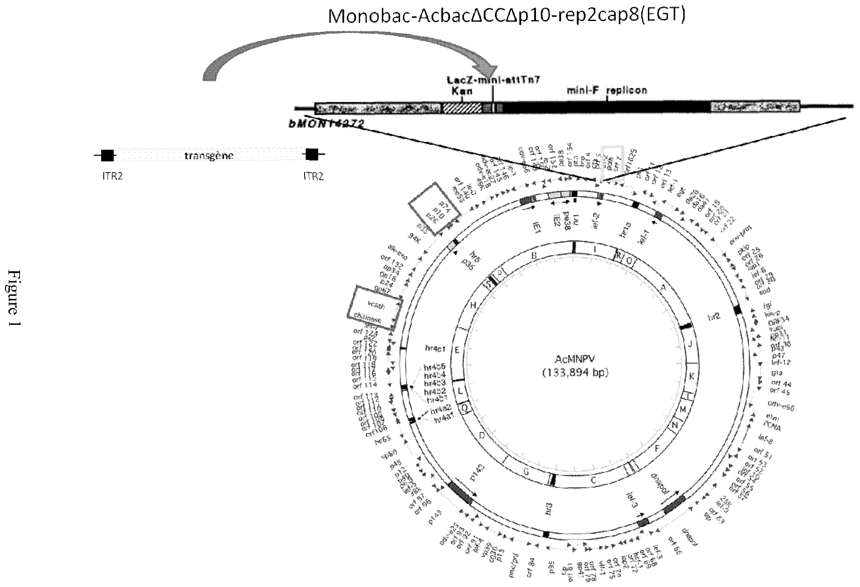

### Figure 1. Système Monobac pour la production de vecteurs rAAV.

Le bacmide d'AcMNPV a été inactivé pour les gènes *chitinase, cathepsine* et *p10*. La cassette d'expression des gènes d'AAV *rep2* et *cap8* a été insérée dans le génome du bacmide au locus ecdysteroid UDP-glucosyltransferase (*egt*), laissant ainsi le site de transposition tn7 du bacmide libre pour l'insertion du génome d'AAVr. Un seul baculovirus permet ainsi de produire des particules d'AAVr en cellules d'insectes.

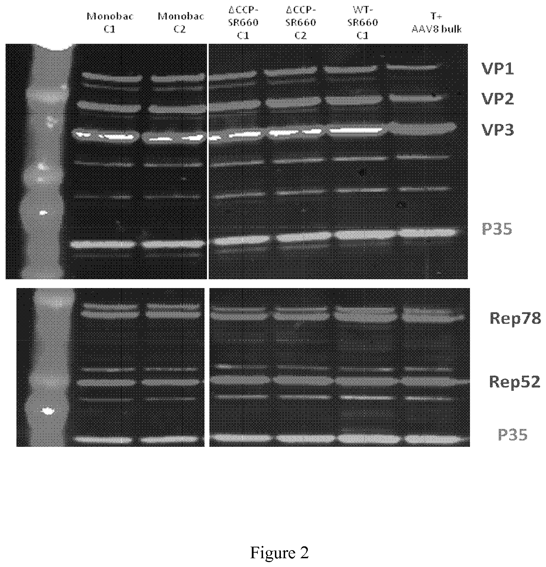

### Figure 2. Etude de l'effet locus pour l'expression des gènes d'AAV rep2 et cap8.

L'expression des protéines d'AAV Rep2 et Cap8 a été suivie par Western blot à partir de baculovirus exprimant ces protéines depuis la cassette d'expression insérée au site Tn7 du bacmide ou au locus *egt,* 3 jours après infection des cellules Sf9. La standardisation de l'expression des protéines est suivie en mesurant l'expression de la protéine baculoviral P35.
Monobac C1 & C2: AcbacΔCCΔp10-rep2cap8(EGT).
ΔCCP-SR660 C1 & C2: AcbacΔCCΔp10-rep2cap8(Tn7).
WT-SR660 C1: AcbacWT.
T+ AAV8 bulk: contrôle positif de production d'AAV8-mSeAP.
ΔCCΔp10: bacmide délété des gènes *cathépsine, chitinase* et *p10.*
Acbac: bacmide dérivé d'AcMNPV

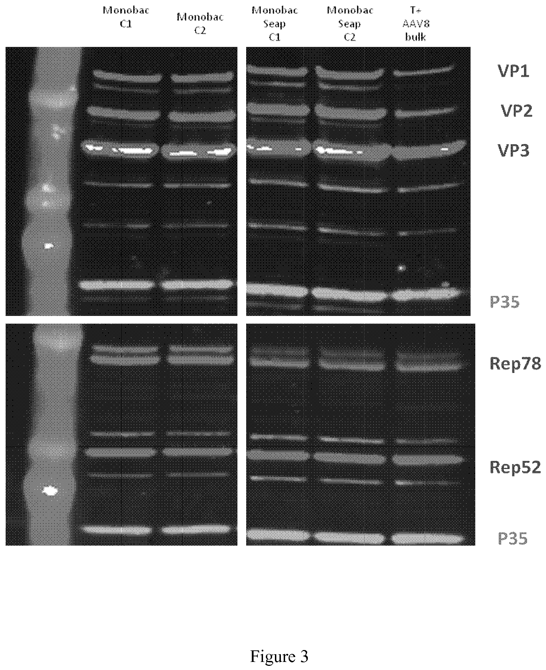

### Figure 3. Etude de l'effet locus pour l'expression des gènes d'AAV rep2 et cap8.

L'expression des protéines d'AAV Rep2 et Cap8 ont été suivies par Western blot à partir de baculovirus exprimant ces protéines depuis la cassette d'expression insérée au site Tn7 du bacmide ou au locus *egt,* 3 jours après infection des cellules Sf9. La standardisation de l'expression des protéines est suivie en mesurant l'expression de la protéine baculoviral P35. Monobac C1 et C2: AcbacΔCCΔp10-rep2cap8(EGT).
Monobac Seap C1 & C2: AcbacΔCCΔp10-rep2cap8(EGT)-mSeAP(Tn7).
T+ AAV8 bulk: contrôle positif de production d'AAV8-mSeAP.

### Figure 4. productivité d'AAVr.

La productivité d'AAVr dans le système monobac a été évaluée. Les résultats sont présentés en titre d'AAVr (vg/ml - vecteur génome/ml). 4 réplicats ont été utilisés par expérience, la barre d'erreur représente l'écart type.

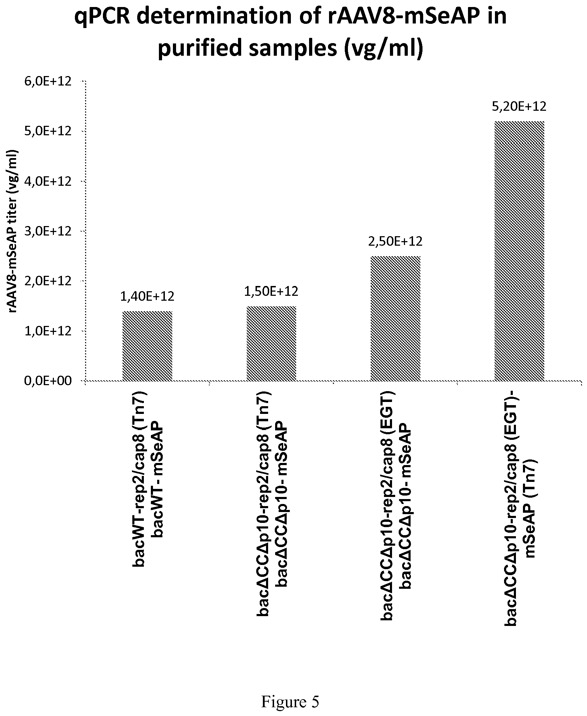

### Figure 5. Productivité d'AAVr après purification par immuno-affinité.

La productivité d'AAVr dans le système monobac a été évaluée après purification. Les résultats sont présentés en titre d'AAVr (vg/ml - vecteur génome/ml).

### Figure 6. Profil protéique des vecteurs AAVr purifiés.

Le profil protéique des vecteurs AAVr a été évalué après purification par immuno-affinité. 5 x 10¹⁰ vg d'AAVr ont été analysés par SDS-PAGE coloré au bleu de Coomassie. (1) AAV8-mSeAP produit avec les baculovirus sauvages. 1^{er} baculovirus bacWT-rep2/cap8 (Tn7), 2^{ème} baculovirus bacWT- mSeAP. (2) AAV8-mSeAP produit avec les baculovirus délétés pour les gènes *chitinase, cathépsine* et *p10*. 1^{er} baculovirus bacΔCCΔp10-rep2/cap8 (Tn7), 2^{ème} baculovirus bacΔCCΔp10- mSeAP. (3) AAV8-mSeAP produit avec les baculovirus délétés pour les gènes *chitinase, cathépsine* et *p10.* 1^{er} baculovirus bacΔCCΔp10-rep2/cap8 (EGT), 2^{ème} baculovirus bacΔCCΔp10- mSeAP. (4) AAV8-mSeAP produit avec le baculovirus délété pour les gènes *chitinase, cathépsine* et *p10*. Un seul baculovirus utilisé bacΔCCΔp10-rep2/cap8 (EGT)-mSeAP (Tn7).

### Description détaillée de l'invention

La production d'un vecteur viral, notamment un vecteur viral de thérapie génique, nécessite l'expression dans une même cellule des nombreuses composantes du vecteur viral. Par exemple, dans le cas de la production d'un AAV recombinant dans une cellule d'insecte, il est nécessaire de produire dans la cellule:
- un génome recombinant d'AAV comprenant une ITR (pour Inverted Terminal Repeat en anglais) 5', et une cassette d'expression d'une séquence nucléotidique hétérologue d'intérêt (au moins une séquence nucléotidique hétérologue d'intérêt sous le contrôle d'un promoteur efficace pour l'expression dudit gène dans une cellule cible définie ci-dessous , et une ITR 3', et
- les produits des gènes *rep* et *cap* d'AAV.

Nous avons développé un système d'expression baculoviral pour faciliter la production de vecteurs viraux, en ce sens qu'un baculovirus unique est utilisé pour infecter les cellules hôtes productrices du vecteur viral.

La présente demande concerne en particulier un génome baculoviral recombinant comprenant une ou plusieurs cassettes d'expression des composantes protéiques nécessaires à la production d'un vecteur viral hétérologue, et un génome recombinant d'un vecteur viral hétérologue (ou génome hétérologue), lesdites cassettes d'expression et ledit génome hétérologue étant insérés dans un ou plusieurs locus choisi dans le groupe constitué des gènes non-essentiels du baculovirus qui peuvent être remplacés par une séquence d'intérêt sans modifier le fonctionnement du baculovirus.

Dans le cadre de la présente invention, les "gènes non-essentiels du baculovirus" sont définis comme des gènes pouvant être inactivés ou éliminés du génome du baculovirus, sans modifier sa capacité à se développer en culture de cellules d'insecte. Ces gènes sont des gènes intervenant spécifiquement dans la production des ODV (Occlusion Derived Virus) ou des gènes nécessaires à la manipulation de l'insecte hôte dans l'environnement, mais non-nécessaire à l'échelle de cellules d'insecte en culture (Cohen et al. 2009 ; Virologica Sinica 24 : 359-414). Dans le cadre de la présent invention, le ou les locus est (sont) choisi(s) dans le groupe constitué des locus polyédrine, *ctx, egt, 39k, orf51, gp37, iap2, p94, p10* et *odv-e56,* ledit locus étant plus particulièrement le locus *egt.*

Dans le cadre de la présente divulgation, on entend par "vecteur viral hétérologue" un vecteur viral qui n'est pas un baculovirus. Le vecteur viral hétérologue peut notamment être un adénovirus, un virus adéno-associé, un rétrovirus, notamment un lentivirus ou un spumavirus, etc. Plus spécifiquement, le génome baculoviral recombinant selon la présente divulgation est susceptible d'être utilisé pour la production de tout type de vecteurs viraux destinés à être utilisés pour introduire une séquence nucléotidique hétérologue dans une cellule, un tissu ou organisme, notamment un vecteur viral à visée thérapeutique chez l'homme ou l'animal (vecteur de thérapie génique).

Dans le cadre de la présente invention, on entend par "cassette d'expression" une combinaison d'éléments nécessaires à l'expression d'un ou plusieurs gènes. Une cassette d'expression contient donc un promoteur adapté pour une expression dans une cellule hôte, en particulier une cellule eucaryote, plus particulièrement une cellule d'insecte, et une séquence de polyadénylation. L'homme du métier a plusieurs cassettes d'expression dans des cellules eucaryotes (en particulier d'insecte ou de mammifère) à sa disposition.

Comme mentionné ci-dessus, le génome baculoviral recombinant selon la présente divulgation peut être destiné à la production de tout type de vecteurs viraux, notamment des vecteurs viraux de thérapie génique. Par exemple, le génome baculoviral recombinant peut comprendre une ou plusieurs cassettes d'expression de protéines virales nécessaires à la production d'un virus adéno-associé, d'un adénovirus, d'un rétrovirus, notamment un lentivirus ou un spumavirus, etc. Les gènes nécessaires à la production de telles particules virales sont bien connus de l'homme du métier, qui saura adapter le présent génome baculoviral recombinant au vecteur viral particulier qu'il souhaite produire (Bagnis, Merten, Mezzina (guest editors) 2005 : Advanced methods for industrial production, purification, and characterization of gene vectors. Gene Ther 12(S1): 1-177).

Selon un mode préféré de réalisation, le génome baculoviral recombinant selon la présente divulgation comprend également un génome recombinant de vecteur viral de thérapie génique, ledit génome recombinant de vecteur viral de thérapie génique étant inséré dans un locus du génome baculoviral tel que décrit ci-dessus. Le génome recombinant de vecteur viral de thérapie génique inséré dans le génome baculoviral dépendra bien entendu du vecteur viral de thérapie génique à produire *in fine.* Ainsi, pour la production d'un AAV, le génome recombinant de vecteur viral de thérapie génique sera un génome recombinant d'AAV comprenant une ITR 5', au moins une séquence nucléotidique hétérologue d'intérêt sous le contrôle d'un promoteur efficace dans la cellule cible de l'AAV recombinant produit, et une ITR 3'. Pour la production d'un lentivirus, le génome recombinant de vecteur viral de thérapie génique sera un génome recombinant de lentivirus comprenant une LTR 5', un site majeur donneur d'épissage, un signal d'empaquetage couvrant la partie 5' du gène Gag, l'élément de réponse à Rev (RRE), l'accepteur d'épissage d'enveloppe, au moins une séquence nucléotidique hétérologue d'intérêt sous le contrôle d'un promoteur efficace dans la cellule cible du lentivirus recombinant produit, et une LTR 3', notamment une LTR 3' modifiée en vue de la génération de vecteurs SIN (lentivirus auto-inactivant - Self-INactivating).

Selon l'invention, le génome baculoviral recombinant décrit est un génome baculoviral pour la production d'un vecteur AAV.

A ce titre, l'invention concerne un génome baculoviral recombinant comprenant une ou plusieurs cassettes d'expression des gènes *rep* et/ou *cap* d'AAV, lesdites une ou plusieurs cassettes d'expression étant insérées dans un locus choisi dans le groupe constitué des locus *egt, ctx, 39k, orf51, gp37, iap2, odv-e56, p10* et *p94* du génome baculoviral. Ce génome baculoviral recombinant contient également un génome recombinant AAV dans un locus choisi parmi les locus *polyédrine, egt, ctx, 39k, orf51, gp37, iap2, odv-e56, p10* et *p94* du génome baculoviral, ledit génome recombinant AAV étant inséré dans un locus différent du(des) locus utilisés pour les gènes *rep* et *cap* d'AAV.

Dans un mode particulier de réalisation, le génome baculoviral recombinant selon l'invention comprend au moins une cassette d'expression des gènes *rep* et/ou *cap* d'AAV dans un locus choisi dans le groupe constitué des gènes non-essentiels du baculovirus qui peuvent être remplacés par une séquence d'intérêt sans modifier le fonctionnement du baculovirus. Ladite au moins une cassette d'expression des gènes *rep* et/ou *cap* d'AAV peut notamment être incluse dans un locus choisi dans le groupe constitué des locus polyédrine, *ctx, egt, 39k, orf51, gp37, iap2, p94, p10* et *odv-e56,* en particulier dans le locus *egt.*

Dans un autre mode particulier de réalisation, le génome baculoviral recombinant selon l'invention comprend en outre un génome recombinant AAV. Le locus d'insertion du génome recombinant AAV est notamment choisi dans le groupe constitué des locus *polyédrine, egt, ctx, 39k, orf51, gp37, iap2, p94, p10* et *odv-e56.* Selon un mode particulier de réalisation, le locus choisi pour le génome recombinant AAV est un locus différent du(des) locus choisi pour la(les) cassette(s) d'expression de *rep* et/ou *cap.* Selon un mode préféré de réalisation, le génome recombinant AAV est inséré dans le locus polyédrine (notamment au niveau du site de recombinaison Tn7).

Selon un premier mode préféré de réalisation, le génome baculoviral recombinant selon l'invention comprend :
- un génome recombinant AAV dans le locus *polyédrine,* et
- une cassette d'expression des gènes *rep*/*cap* d'AAV dans un locus choisi dans le groupe constitué des locus *ctx, egt, 39k, orf51, gp37, iap2, p94, p10* et *odv-e56,* en particulier le locus *egt.*

Selon un deuxième mode préféré de réalisation, le génome baculoviral recombinant selon l'invention comprend :
- un génome recombinant AAV dans le locus *p94,*
- une cassette d'expression des gènes *rep*/*cap* d'AAV dans un locus choisi dans le groupe constitué des locus *polyédrine, ctx, egt, 39k, orf51*, *gp37, iap2, p10* et *odv-e56,* en particulier le locus *egt.*

Les cassettes d'expression des gènes *rep* et *cap* sont notamment choisies en fonction du gène d'intérêt à exprimer à partir de l'AAV recombinant produit et du type de cellules à transduire avec ledit AAV. L'homme du métier est en mesure de sélectionner et produire les cassettes d'expression adéquates sur la base de ses connaissances générales. A titre de promoteurs particuliers, on peut citer les promoteurs baculoviraux précoces/tardifs tels que gp64, ou des promoteurs baculoviraux très tardifs tels que les promoteurs des gènes polyédrine (P_{Ph}) et p10 (Pₚ₁₀), si le baculovirus est destiné à infecter des cellules d'insecte. Les promoteurs très précoces comme IE1 (Immediate Early 1) sont aussi utilisables dans le cadre de l'invention, avec possibilité de fonctionner dans des cellules de mammifères par exemple. A titre de séquences de polyadénylation, on peut notamment citer les séquences de polyadénylation de la tryptophane hydroxylase (Tph), ou des séquences de polyadénylation de gènes baculoviraux.

Les gènes *rep* et *cap* peuvent être sélectionnés selon le type d'AAV recombinant dont la production est souhaitée. Ils peuvent être choisis parmi les gènes *rep* et *cap* d'AAV de tout sérotype. Les gènes *rep* et *cap* peuvent être inclus dans des cassettes d'expression différentes ou dans une cassette unique. Selon un mode préféré de réalisation, une cassette d'expression unique est utilisée pour l'expression des gènes *rep* et *cap.* Selon une variante de réalisation, la cassette d'expression des gènes *rep*/*cap* d'AAV comprend lesdits gènes en orientation inverse. Ces gènes en orientation inverse peuvent notamment être chacun sous le contrôle de promoteurs différents, notamment de promoteurs baculoviraux différents, en particuliers des promoteurs très tardif différents. Par exemple, les promoteurs très tardifs en question peuvent notamment correspondre aux promoteurs baculoviraux P_{P10} et P_{Ph}. Selon un mode de réalisation particulièrement préféré, la cassette d'expression de *rep* et *cap* est produite et insérée dans le génome baculoviral recombinant de l'invention selon les procédures décrites dans Smith et al., 2009; Mol. Ther. 17: 1888-1896. Selon un mode particulier, la cassette d'expression correspond à celle décrite dans les exemples ci-dessous, permettant l'expression des produits des gènes *rep2* et *cap8,* et donc la production de vecteur AAV recombinants de type AAV8, comprenant des protéines de capside de l'AAV8, et donc présentant le tropisme de ce sérotype particulier. Cette cassette d'expression est préférentiellement introduite dans le génome baculoviral par transposition, selon des procédures bien connues dans le domaine, notamment en utilisant le système Bac-to-Bac commercialisé par la société Invitrogen.

Selon un mode particulier de réalisation, la cassette d'expression des gènes *rep*/*cap* d'AAV est insérée dans le locus *egt,* en particulier selon le procédé décrit dans les exemples.

Le génome recombinant d'AAV comprend une ITR (pour Inverted Terminal Repeat en anglais) 5', au moins une séquence nucléotidique hétérologue d'intérêt sous le contrôle d'un promoteur efficace dans la cellule cible de l'AAV recombinant produit, et une ITR 3'. Les ITRs peuvent être dérivées de tout sérotype d'AAV connu de l'homme du métier.

Par "séquence nucléotidique hétérologue d'intérêt", on entend une séquence nucléotidique qui n'est pas un gène baculoviral ou un gène d'AAV et qui, lorsqu'elle est exprimée dans une cellule d'intérêt (aussi dite "cellule cible"), permet d'obtenir un effet souhaité. La séquence nucléotidique hétérologue peut notamment coder une protéine, un ARN interférant, un ARN antisens, un microARN ou snRNA. L'effet souhaité peut notamment correspondre à l'expression de la séquence nucléotidique hétérologue dans des cellules ou tissus d'un patient. Ainsi, la séquence nucléotidique hétérologue peut notamment être administrée afin d'obtenir un effet thérapeutique. A ce titre, l'AAV recombinant produit sera utilisable comme vecteur de thérapie génique. La séquence nucléotidique hétérologue pourra ainsi permettre la production d'une protéine thérapeutique, par exemple une protéine déficiente dans une maladie, ou un ARN interférant dans des cellules nécessitant la diminution de l'expression d'une protéine anormale, ou un snRNA (small nuclear RNA) impliqué dans l'élimination d'un exon muté responsable de la non-fonctionnalité d'une protéine.

La cellule cible correspond à toute cellule dans laquelle l'expression de la séquence nucléotidique hétérologue peut avoir un intérêt, notamment thérapeutique. Le promoteur présent dans le génome AAV sera donc un promoteur permettant l'expression de la séquence nucléotidique hétérologue dans la cellule cible. Ce promoteur peut être constitutif, inductible ou spécifique d'une cellule ou d'un tissu particulier.

Bien entendu, l'homme du métier est familier avec les concepts de la thérapie génique et adaptera les éléments constitutifs de la présente invention à ses besoins.

Le génome baculoviral recombinant peut également comprendre toute séquence d'AAV permettant une amélioration de la quantité ou de la qualité de l'AAV recombinant. On peut citer à ce titre l'insertion, dans l'un des locus cités ci-dessus, du gène codant pour la protéine AAP (ou Assembly Activating Protein en anglais - Sonntag et al., Proc Natl Acad Sci USA. 2010 Jun 1;107(22):10220-5).

Selon une seconde variante de l'invention , le génome baculoviral recombinant décrit est un génome baculoviral pour la production d'un vecteur lentiviral.

A ce titre, la présente divulgation concerne également un génome baculoviral recombinant comprenant une ou plusieurs cassettes d'expression des gènes *gag*/*pol*, *rev* de lentivirus (par ex. en provenance d'HIV-1) et/ou *env* choisi selon le tropisme voulu, mais de préférence la protéine G en provenance du virus de la stomatite vesiculaire (*VSV-G*), lesdites une ou plusieurs cassettes d'expression étant insérées dans un locus choisi dans le groupe constitué des locus *egt, ctx, 39k, orf51*, *gp37, iap2, odv-e56, p10 et p94* du génome baculoviral. Ce génome baculoviral recombinant contient également de préférence un génome recombinant de lentivirus dans un locus choisi parmi les locus *polyédrine, egt, ctx, 39k, orf51*, *gp37, iap2, odv-e56, p10* et *p94* du génome baculoviral, ledit génome recombinant de lentivirus étant inséré dans un locus différent du(des) locus utilisés pour les gènes *gag*/*pol* et *rev* de lentivirus ainsi que le gène *env.*

Dans un mode particulier de réalisation, le génome baculoviral recombinant selon la présente divulgation comprend au moins une cassette d'expression des gènes *gag*/*pol* et *rev* de lentivirus ainsi que le gène *env* dans un locus choisi dans le groupe constitué des gènes non-essentiels du baculovirus qui peuvent être remplacés par une séquence d'intérêt sans modifier le fonctionnement du baculovirus. Ladite au moins une cassette d'expression des gènes gènes *gag*/*pol* et *rev* de lentivirus ainsi que le gène *env* peut notamment être incluse dans un locus choisi dans le groupe constitué des locus polyédrine, *ctx, egt, 39k, orf51*, *gp37, iap2, p94, p10* et *odv-e56.*

Dans un autre mode particulier de réalisation, le génome baculoviral recombinant selon la présente divulgation comprend en outre un génome recombinant de lentivirus. Le locus d'insertion du génome recombinant de lentivirus est notamment choisi dans le groupe constitué des locus *polyédrine, egt, ctx, 39k, orf51*, *gp37, iap2, p94, p10* et *odv-e56.* Selon un mode particulier de réalisation, le locus choisi pour le génome recombinant de lentivirus est un locus différent du(des) locus choisi pour la(les) cassette(s) d'expression de *gag*/*pol, rev* et/ou *env.* Selon un mode préféré de réalisation, le génome recombinant de lentivirus est inséré dans le locus *polyédrine* (notamment au niveau du site de recombinaison Tn7).

Selon un premier mode préféré de réalisation, le génome baculoviral recombinant selon la présente divulgation comprend :
- un génome recombinant de lentivirus dans le locus *polyédrine,* et
- une cassette d'expression des gènes *gag*/*pol* et *rev* de lentivirus ainsi que le gène *env* dans un locus choisi dans le groupe constitué des locus *ctx, egt, 39k, orf51, gp37, iap2, p94, p10* et *odv-e56.*

Selon un deuxième mode préféré de réalisation, le génome baculoviral recombinant selon la présente divulgation comprend :
- un génome recombinant de lentivirus dans le locus *p94,*
- une cassette d'expression des gènes *gag*/*pol* et *rev* de lentivirus ainsi que le gène *env* dans un locus choisi dans le groupe constitué des locus *polyédrine, ctx, egt, 39k, orf51, gp37, iap2, p10* et *odv-e56.*

Les cassettes d'expression des gènes *gag*/*pol* et *rev* de lentivirus ainsi que le gène *env* sont notamment choisies en fonction du gène d'intérêt à exprimer à partir du lentivirus recombinant produit et du type de cellules à transduire avec ledit lentivirus. L'homme du métier est en mesure de sélectionner et produire les cassettes d'expression adéquates sur la base de ses connaissances générales. A titre de promoteurs particuliers, on peut citer les promoteurs baculoviraux précoces/tardifs tels que gp64, ou des promoteurs baculoviraux très tardifs tels que les promoteurs des gènes *polyédrine* (P_{Ph}) et p10 (Pₚ₁₀), si le baculovirus est destiné à infecter des cellules d'insecte. Les promoteurs très précoces comme IE1 (Immediate Early 1) sont aussi utilisables dans le cadre de l'invention, avec possibilité de fonctionner dans des cellules de mammifères par exemple. A titre de séquences de polyadénylation, on peut notamment citer les séquences de polyadénylation de la tryptophane hydroxylase (Tph), ou des séquences de polyadénylation de gènes baculoviraux.

Par "séquence nucléotidique hétérologue d'intérêt", on entend une séquence nucléotidique qui n'est pas un gène baculoviral ou un gène du lentivirus et qui, lorsqu'elle est exprimée dans une cellule d'intérêt (aussi dite "cellule cible"), permet d'obtenir un effet souhaité. La séquence nucléotidique hétérologue peut notamment coder une protéine, un ARN interférant, un ARN antisens, un microARN ou snRNA. L'effet souhaité peut notamment correspondre à l'expression de la séquence nucléotidique hétérologue dans des cellules ou tissus d'un patient. Ainsi, la séquence nucléotidique hétérologue peut notamment être administrée afin d'obtenir un effet thérapeutique. A ce titre, le lentivirus recombinant produit sera utilisable comme vecteur de thérapie génique. La séquence nucléotidique hétérologue pourra ainsi permettre la production d'une protéine thérapeutique, par exemple une protéine déficiente dans une maladie, ou un ARN interférant dans des cellules nécessitant la diminution de l'expression d'une protéine anormale, ou un snRNA (small nuclear RNA) impliqué dans l'élimination d'un exon muté responsable de la non-fonctionnalité d'une protéine.

La cellule cible correspond à toute cellule dans laquelle l'expression de la séquence nucléotidique hétérologue peut avoir un intérêt, notamment thérapeutique. Le promoteur présent dans le génome de lentivirus sera donc un promoteur permettant l'expression de la séquence nucléotidique hétérologue dans la cellule cible. Ce promoteur peut être constitutif, inductible ou spécifique d'une cellule ou d'un tissu particulier.

Bien entendu, l'homme du métier est familier avec les concepts de la thérapie génique et adaptera les éléments constitutifs de la présente invention à ses besoins.

Le génome baculoviral recombinant peut être dérivé de tout baculovirus comprenant le locus *polyédrine* (ou équivalent), et au moins un ou plusieurs des autres locus mentionnés ci-dessus (ou un ou plusieurs locus équivalents).
Selon un mode particulier de réalisation, le génome baculoviral recombinant selon l'invention est un bacmide. Dans le cadre de la présente invention, un "bacmide" est un génome baculoviral comprenant des éléments génétiques permettant le maintien et l'amplification d'un génome baculoviral dans une cellule procaryote. Il peut notamment comprendre une origine de réplication bactérienne, un gène de sélection, notamment un gène de résistance à un antibiotique tel que la kanamycine, et un site de clonage par recombinaison tel que le « Tn7 » insérés dans un locus baculoviral tel le locus *polyédrine*. Selon une variante, le bacmide recombinant selon l'invention comprend une origine de réplication, un gène de résistance à la kanamycine et un site de clonage par recombinaison Tn7 dans le locus *polyédrine* (notamment le bacmide AcMNPV décrit dans Luckow et al., 1993; J. Virol. 67:4566-4579). Ainsi, le génome baculoviral recombinant peut correspondre à une séquence capable de se répliquer dans des cellules d'insecte et dans un organisme procaryote tel que *E. coli.* En particulier, tout génome baculoviral comprenant une construction ADN permettant le maintien du génome dans un organisme procaryote, en particulier un réplicon BAC, peut être utilisé. Selon un aspect préféré, le génome baculoviral de l'invention est dérivé d'un squelette AcMNPV ou SpliNPV. La position des locus génétiques *ctx, egt, 39k, orf51, gp37, iap2* et *odv-e56* dans la séquence de référence AcMNPV (numéro d'accès NC001623) sont notamment décrits dans le tableau 1 de la demande internationale WO 2010/055292. En ce qui concerne les locus génétiques *p94* et *p10,* leur position est présentée en Figure 1 (séquence de référence AcMNPV - numéro d'accès NC001623).

Selon un mode particulier de réalisation, le génome baculoviral recombinant est dérivé d'AcMNPV et comprend une délétion des gènes *chitinase, cathépsine* et *p10.* Ainsi, selon ce mode de réalisation, l'invention concerne un génome baculoviral AcMNPV recombinant dépourvu des gènes *chitinase, cathépsine* et *p10* et comprenant une cassette d'expression des gènes *rep*/*cap* d'AAV dans un locus choisi dans le groupe constitué des locus *egt, polyédrine, ctx, 39k, orf51, gp37, iap2* et *odv-e56, p10 et p94,* en particulier le locus *egt.*
Dans une variante de ce mode de réalisation, le bacmide comprend par ailleurs un génome recombinant AAV dans le locus *polyédrine* (Tn7 du bacmide).
Dans une autre variante de ce mode de réalisation, le génome baculoviral comprend par ailleurs un génome recombinant AAV dans le locus *p94.*

La délétion des gènes *chitinase, cathépsine* et *p10* dans le squelette baculoviral permet une production hautement efficace de vecteurs AAV, permettant une dégradation protéolytique réduite, en particulier des protéines VP1 et VP2, et donc une augmentation de l'infectivité et de l'efficacité *in vivo.* La délétion de ces gènes peut être réalisée selon toute procédure connue de l'homme du métier. On utilisera plus particulièrement le procédé décrit dans les exemples mettant en oeuvre le processus de recombinaison homologue (Datsenko et Wanner, 2000; Proc Natl Acad Sci USA. 97(12):6640-6645) dans des bactéries *E. coli* contenant le bacmide à modifier (notamment le bacmide AcMNPV décrit dans Luckow et al., 1993; J. Virol. 67:4566-4579).

Selon un mode particulier de réalisation, les gènes *chi*/*v-cath* (nucleotides 105282-107954 selon la carte génétique d'AcMNPV (Ayres *et al*., 1994)) et *p10* (nucleotides 118839-119121) sont délétés.

Selon une variante du mode de réalisation comprenant une délétion des gènes *chitinase, cathépsine* et *p10*, la délétion du gène *p10* n'est pas accompagnée d'une délétion du promoteur du gène *p10.* En d'autres termes, le génome baculoviral recombinant déficient pour les gènes *chitinase, cathépsine* et *p10* comprend un promoteur P_{P10} fonctionnel (correspondant aux nucléotides 118739-118836 de la carte génétique d'AcMNPV).

Selon une autre variante du mode de réalisation comprenant une délétion des gènes *chitinase, cathépsine* et *p10*, la délétion du gène p10 et accompagnée d'une délétion des gènes *p26* et *p74* adjacents. Selon une aspect particulier de ce mode de réalisation, la délétion des gènes *p26*, *p10 et p74* correspond aux nucléotides 118044-121072 de la carte génétique d'AcMNPV (Ayres et al., 1994).

Selon un deuxième aspect, l'invention concerne un baculovirus recombinant comprenant un génome correspondant au génome baculoviral décrit ci-dessus. Selon un mode préféré de réalisation, le un baculovirus recombinant comprend un génome correspondant au bacmide recombinant décrit ci-dessus

Selon un troisième aspect, l'invention concerne un procédé pour produire un baculovirus selon l'invention, comprenant la culture d'une cellule procaryote contenant le bacmide recombinant défini ci-dessus dans des conditions adaptées à la production d'un baculovirus. Ces conditions sont bien connues de l'homme du métier (Smith et al., 2009, supra; Luckow et al., 1993, supra).

Selon un quatrième aspect, l'invention concerne une cellule eucaryote ou procaryote contenant le génome baculoviral défini ci-dessus. La cellule peut notamment être une cellule eucaryote de mammifère ou d'insecte, en particulier une cellule d'insecte qui a été infectée par un baculovirus recombinant tel que défini ci-dessus.

Parmi les cellules d'insecte, on préférera celles dérivées des lignées *Spodoptera frugiperda* ou *Trichoplusia ni*, par exemple les cellules Sf21, Sf9, High five ou TN 5B1-4. Parmi les cellules de mammifère, ou pourra notamment citer la lignée HEK293, connue pour être susceptible d'être infectée par un baculovirus. Dans ce dernier cas, le(s) promoteur(s) présent(s) dans la cassette d'expression des gènes *rep* et *cap* sera(seront) adapté(s) pour une expression dans des cellules de mammifère. On peut notamment citer le promoteur CMV et d'autres promoteurs bien connu dans le domaine.

Selon un cinquième aspect, l'invention concerne par ailleurs un procédé pour produire un vecteur viral, notamment de thérapie génique, comprenant la mise en culture du baculovirus recombinant défini ci-dessus avec une cellule, notamment une cellule d'insecte ou une cellule de mammifère, dans des conditions permettant l'infection de la cellule par le baculovirus et la production dudit vecteur viral.

En ce qui concerne la production des vecteurs enveloppés (tels que les vecteurs rétroviraux), il s'agit d'une cellule mammifère (par ex. une cellule HEK293).

Le procédé selon l'invention est un procédé pour produire un AAV recombinant comprenant la mise en culture du baculovirus recombinant défini ci-dessus avec une cellule, notamment une cellule d'insecte (e.g. une cellule Sf21, Sf9, High five ou TN 5B1-4), susceptible d'être infectée par ledit baculovirus, dans des conditions permettant l'infection de la cellule par le baculovirus et la production dudit AAV recombinant. Ainsi, il est possible de produire un AAV recombinant à partir d'une infection avec un baculovirus unique. Des conditions particulières, non limitatives, de production d'un AAV recombinant selon l'invention sont notamment décrites dans les exemples. Bien entendu, l'homme du métier est en mesure d'adapter ces conditions de production sur la base de ses connaissances générales (Smith et al., supra).

Grâce à ce procédé, on obtient une production d'AAV recombinant au moins 5 fois supérieure à celle obtenue avec un système nécessitant une infection avec deux baculovirus tel que le système décrit par Smith et al.

Selon un autre mode particulier de réalisation, est décrit ici un procédé pour la production d'un lentivirus recombinant, comprenant la mise en culture du baculovirus recombinant défini ci-dessus avec une cellule de mammifère (par exemple une cellule HEK293), susceptible d'être infectée par ledit baculovirus, dans des conditions permettant l'infection de la cellule par le baculovirus et la production dudit lentivirus recombinant.

Les exemples ci-dessous sont fournis pour illustrer l'invention.

### Exemples

### MATERIELS ET METHODES:

### Séquences

**Table 1: séquences d'amorces**

| **Amorce** | **Séquence 5' to 3'** | **Utilisation*** |
|---|---|---|
| EGT-lox-F | | insertion de rep2 / cap8 dans le bacmide AcMNPV au niveau du locus EGT (11634 - 12486) |
| EGT-SV40-R | | insertion de rep2 / cap8 dans le bacmide AcMNPV au niveau du locus EGT (11634 - 12486) |
| EGT-Control 150F | ATGACTATTCTCTGCTGGC (SEQ ID NO:3) | Vérification |
| EGT-Control 150R | ATTGGCCGTGTTTCCTAC (SEQ ID NO:4) | Vérification |
| M13 PUC F | CCAGTCACGACGTTGTAAAACG (SEQ ID NO:5) | Vérification des bacmides transposés |
| M13 PUC R | AGCGGATAACAATTTCACACAGG (SEQ ID NO:6) | Vérification des bacmides transposés |
| Genta | AGCCACCTACTCCCAACATC (SEQ ID NO:7) | Vérification des bacmides transposés |
| CC-KO-F | | inactivation des gènes chitinase/cathepsine nt 105771-107700 |
| CC-KO-R | | inactivation des gènes chitinase/cathepsine nt 105771-107700 |
| chitinase-105625F | CGCGGCCGTACATGGCGACGCCCA (SEQ ID NO:10) | Vérification |
| cathepsin-107849R | GTTTTTAAAGGTCCAATATGGAATG (SEQ ID NO:11) | Vérification |
| p10-KO-F | | inactivation de la séquence codant p10 (du codon start au codon stop) nt 118839 - 119121 |
| P10-KO-R | | inactivation de la séquence codant p10 (du codon start au codon stop) nt 118839 - 119121 |
| p10-118725-F | CCGGGACCTTTAATTCAACCCAACA (SEQ ID NO:14) | Vérification |
| p10-119259-R | CAGCATTTGTTATACACACAGAACT (SEQ ID NO:15) | Vérification |

| | | |
|---|---|---|
| * numérotation selon Ayres et al., Virology. 1994 Aug 1;202(2):586-605. | | |

### Délétion de gènes du baculovirus

La délétion de la *cathépsine* et de la *chitinase* dans le bacmide AcMNPV sauvage a été réalisée à partir de la souche *E. coli* DH10Bac contenant le bacmide AcMNPV (Luckow et al., 1993, cf supra) et transformée par pKD46 (Datsenko et Wanner, 2000, PNAS Vol 97 (12) pages 6640-6645). Un produit de PCR nécessaire pour l'inactivation des gènes *cathepsine*/*chitinase* a été généré au moyen des amorces CC-KO-F et CC-KO-R (tableau 1). L'inactivation des gènes a été réalisée suivant la méthode décrite par Marek et al., 2011 et évaluée en utilisant les amorces chitinase-105625F et cathepsin-107849R (tableau 1). La suppression du gène marqueur *CAT* du bacmide *cathepsine*/*chitinase* null (AcbacΔCCΔcat) a été réalisée selon la méthode décrite par Marek et al. (Marek et al., 2011, Biotechnol Bioeng, Vol 108 (5) pages 1056 -67) et vérifiée par PCR et séquençage, en utilisant les amorces mentionnées ci-dessus. L'inactivation de la séquence codante *p10* dans AcbacΔCCΔcat a été réalisée de la même manière, en générant un produit de PCR avec les amorces p10-KO-F/p10-KO-R (tableau 1). La vérification de l'inactivation correcte du gène a été réalisée par PCR et séquençage avec la paire d'amorces p10-118725-F/ p10-119259-R (tableau 1). La dernière inactivation de gène permet la production du bacmide *cathepsine*/*chitinase*/*p10* null (AcbacΔCCΔp10).

### Insertion de la cassette rep2/cap8 au locus egt du bacmide d'AcMNPV

L'insertion de la cassette d'expression des gènes d'AAV *rep2* et *cap8* au locus *egt,* a été réalisée dans le génome du bacmide, déjà inactivé pour les gènes *chitinase, cathépsine* et *p10.* La cassette d'expression *rep2*/*cap8* a été amplifiée par PCR depuis le plasmide pSR660, grâce aux amorces EGT-lox-F et EGT-SV40-R. L'insertion de ce produit PCR dans le génome du bacmide a été effectuée suivant la méthode décrite par Marek et al., 2011. Brièvement, la cassette d'expression *rep2*/*cap8* a été couplée à un gène de résistance à l'antibiotique Chloramphénicol, bordé des séquences *lox66* et *lox71* (Suzuki et al., 2005 ; appl Environ Microbiol. 71(12) 8472-8480). Ces deux éléments génétiques permettent par la suite l'élimination de ce gène de résistance, par l'action de la Cre recombinase, qui peut alors être utilisé pour des enchainements successifs de délétions ou d'insertions de gènes dans le génome du bacmide. La cassette décrite supra est alors amplifiée par PCR grâce aux amorces EGT-lox-F et EGT-SV40-R. Ce produit PCR purifié sur gel d'agarose et traité par l'enzyme de restriction DpnI. Il est alors utilisé pour transformer chimiquement des bactéries compétentes contenant le bacmide, déjà inactivé pour les gènes *chitinase, cathépsine* et *p10,* le plasmide pKD46 (Datsenko et Wanner 2000). L'expression des gènes de l'opéron Red du phage lambda codés par le plasmide pKD46 est induite par l'ajout de L-arabinose à 0,1% (Masse/Volume). Les bactéries recombinantes résistantes au Chloramphenicol sont analysées par PCR pour vérifier l'insertion de la cassette d'expression *rep2*/*cap8* dans le génome du bacmide déjà inactivé pour les gènes *chitinase, cathépsine* et *p10,* au locus *egt* a été vérifié par PCR en utilisant les amorces EGT-Control 150F et EGT-Control 150R (Tableau 1). Cette insertion a permis l'obtention du bacmide Monobac-AcbacΔCCΔp10-rep2cap8(EGT)

### Insertion de la cassette rep2/cap8 et du génome recombinant d'AAVr dans le bacmide au site Tn7.

Les bactéries E.coli DH10b contenant soit le bacmide AcbacΔCCΔp10, ou le bacmide Monobac-AcbacΔCCΔp10-rep2cap8(EGT) ont été transformées par le plasmide pMON7124 (Luckow et al., 1993). Dans ces bactéries, ainsi que dans les bactéries DH10bac contenant le bacmide Acbac sauvage (Luckow et al., 1993), ont été insérés par recombinaison (Luckow et al. 1993), soit le génome recombinant d'AAV codant pour le gène rapporteur de la *phosphatase alcaline sécrétée murine* (ou *murine secreted alkaline phosphatase* (mSeAP) en anglais) sous le contrôle du promoteur CMV et entouré des ITR d'AAV ; soit la cassette d'expression des gènes d'AAV *rep2* et *cap8.* Ces recombinaisons ont respectivement été faites à partir des plasmides pFBD-mSeAP et pFBD-SR660. Ces recombinaisons ont permis de générer les bacmides :
- AcbacWT-rep2/cap8(Tn7) ; AcbacΔCCΔp10-rep2/cap8(Tn7) permettant l'expression des gènes *rep2* et *cap8* depuis le site Tn7.
- AcbacWT-mSeAP(Tn7) ; AcbacΔCCΔp10-mSeAP(Tn7) contenant le génome d'AAVr codant pour le gène mSeAP au site Tn7 du bacmide.
- AcbacΔCCΔp10-rep2/cap8(EGT)mSeAP(Tn7) permettant l'expression des genes *rep2* et *cap8* depuis le locus *egt* et contenant le génome d'AAVr codant pour le gène mSeAP au site Tn7 du bacmide.
- Enfin, le bacmide AcbacΔCCΔp10-rep2/cap8(EGT) permet l'expression des gènes *rep2* et *cap8* depuis le locus *egt* mais n'a pas reçu le génome recombinant d'AAVr-mSeAP au site Tn7.

### Lignée cellulaire, baculovirus et production d'AAVr

Les cellules Sf9 sont entretenues à 27°C dans du milieu SF900II (Invitrogen) dans des spinners Bellco d'un litre. Les baculovirus sont générés suivant le protocole Bac-to-Bac d'Invitrogen, puis amplifiés dans cultures en suspension de cellules Sf9, dans des spinners Bellco de 100ml. Pour produire les vecteurs AAVr les cellules Sf9 sont infectées avec un ou deux baculovirus codant pour les gènes d'AAV *rep2* et *cap8* et contenant la cassette mSeAP du génome d'AAVr. 70ml de cellules Sf9 à une densité de 10⁶cellules/ml sont donc infectées à une MOI de 0,1 pour produire les vecteurs AAVr. 96 heures après la production, un aliquot est prélevé pour effectuer les titrations des vecteurs AAVr, le reste est conservé à -80°C.

### Purification et caractérisation des vecteurs AAVr.

Les vecteurs AAVr sont purifiés depuis l'ensemble de la culture cellulaire sur une colonne d'immuno-affinité AVB sepharose (GE Healthcare) suivant les recommandations de Smith et al., 2009. Les vecteurs AAVr purifiés sont analysés (5 x 10⁺¹⁰ vg) sur gel de poly-acrylamide « SDS-PAGE » (système NuPAGe Invitrogen). Après migration le gel est coloré au bleu de coomassie.

### Détermination des titres en génomes viraux des vecteurs AAVr.

Afin de déterminer le titre en génomes viraux (vg) des vecteurs AAVr produits, une extraction d'ADN viral est effectuée directement depuis l'ensemble de la culture cellulaire, ou bien depuis des échantillons de vecteurs AAVr purifiés. Cette extraction est effectuée en utilisant le système MagNA Pure DNA and viral NA small volume kit (MagNA Pure 96, Roche). Une PCR quantitative en temps réel est ensuite effectuée, avec des amorces hybridant les ITRs de l'AAV. La quantification absolue est effectuée par rapport à un plasmide de référence contenant les régions amplifiées par PCR quantitative et dont le nombre de copies est connu. Les titrations sont effectuées au même moment, sur la même plaque et dans les mêmes conditions et par un opérateur indépendant, afin de réduire au maximum la variabilité expérimentale.

### Détection des protéines par Western Blot

Les échantillons de culture contenant le vecteurs AAVr ou des échantillons purifiés, sont analysés par Western blot afin des détecter la présence des protéines VP et des protéines Rep d'AAV. La révélation des protéines est effectué suivant la méthode développée par LI-COR, avec un appareil Odyssey et le logiciel Odyssey 2.1. Les anticorps primaires utilisés sont, l'anti VP IgG1 clone B1 de souris (Progen) pour la détection des protéines VP et l'anti Rep IgG1 de souris clone 303.9 (Progen) pour la détection des Rep. Ces anticorps sont utilisés respectivement à une dilution 1/250^{ème} et 1/100^{ème} dans du tampon « infrared imaging system blocking buffer », LI-COR. Les anticorps secondaires de chèvre utilisés sont dirigés contre les anticorps primaires et couplés au fluorochrome « Dye 680 » de LI-COR. La protéine de baculovirus P35 est également détectée par un anticorps primaire spécifique et un anticorps secondaire couplé au « Dye 800 » de LI-COR.

### Analyse statistique

La significativité statistique des différences observées pour les titres d'AAVr obtenu pendant la production a été analysée. Un test de Fisher a été réalisé afin de déterminer l'égalité de deux variances. Un test de Student a ensuite été mis en oeuvre. Le logiciel Excel a été utilisé pour ces analyses.

### RESULTATS

### Génération d'un baculovirus exprimant au locus egt, les gènes d'AAV rep2 et cap8.

Pour insérer les gènes d'AAV *rep2* et *cap8* dans le génome du bacmide d'AcMNPV, un génome de bacmide déjà modifié a été utilisé. Nous avons préalablement montré que l'utilisation d'un bacmide inactivé pour les gènes *chitinase, cathépsine* et *p10* réduisait la protéolyse de la capside d'AAVr pour certains serotypes. L'insertion des gènes d'AAV *rep2* et *cap8* au locus *egt* a donc été effectuée dans le bacmide AcbacΔCCΔp10, suivant la méthodologie développée par Marek et al., 2011 (Figure 1.). Cette insertion a été vérifiée par PCR et séquençage. Elle a aboutit à la création du bacmide AcbacΔCCΔp10-rep2cap8(EGT). Ce bacmide dispose toujours du site classique de clonage par recombinaison, le Tn7 (Luckow et al., 1993), dans lequel a pu être inséré le génome d'AAVr exprimant le gène de la mSeAP et générant ainsi la construction AcbacΔCCΔp10-rep2cap8(EGT)SeAP(Tn7).
Parallèlement les bacmides contrôles ont été générés, avec l'insertion, au site Tn7, du génome d'AAVr ou de la cassette d'expression des gènes d'AAV *rep2* et *cap8.* Ces bacmides contrôles ont été générés sur un fond génétique non modifié ou sur le fond génétique inactivé pour les gènes *chitinase, cathépsine* et *p10.*

Après recombinaison, les ADN de bacmide ont été extraits et purifiés suivant le protocole Bac-to-Bac (Invitrogen) et l'absence de bacmide non recombinant a été vérifié par PCR. Les bacmides ont été transfectés dans les cellules Sf9, purifiés sur plaque par plage de lyse et amplifiés. Aucune différence en termes de titre baculoviral (pfu/ml) n'a été observé entre toutes les constructions.

### L'insertion des gènes d'AAV rep2 et cap8 au locus egt permet leur expression.

Tout d'abord, la capacité à générer le baculovirus AcbacΔCCΔp10-rep2cap8(EGT) en cellules Sf9, sans modifier le titre viral, confirme la possibilité d'insérer une cassette d'expression génique au locus *egt* sans modifier la viabilité du baculovirus, comme précédemment observé par Noad et al., 2009.

Le niveau d'expression des protéines peut être modifié suivant le site d'insertion du gène dans le génome du baculovirus (Noad et al., 2009). Le locus *egt* a donc été choisi pour insérer la cassette d'expression *rep2*/*cap8* sur la base d'une équivalence d'expression en comparaison à l'expression dirigée depuis le site Tn7 (Noad et al., 2009).

Comme observé dans la figure 2, les protéines Rep et VP d'AAV sont exprimées à des niveaux comparables, que la cassette d'expression soit insérée au locus *egt* comme dans la construction AcbacΔCCΔp10-rep2cap8(EGT) (Figure 2, MB C1 et C2) ou au site Tn7 pour les constructions AcbacΔCCΔp10 et AcbacWT (Figure 2, ΔCCP-SR660 C1 et C2 ; WT-SR660 C1 et C2).

Après insertion du génome d'AAVr mSeAP au site Tn7 du bacmide (AcbacΔCCΔp10-rep2cap8(EGT)SeAP(Tn7)), l'expression des gènes *rep2* et *cap8* reste équivalente à celle observée pour ce même baculovirus ne contenant pas le génome de l'AAVr (Figure 3).

### La production d'AAVr par un seul baculovirus améliore la productivité cellulaire.

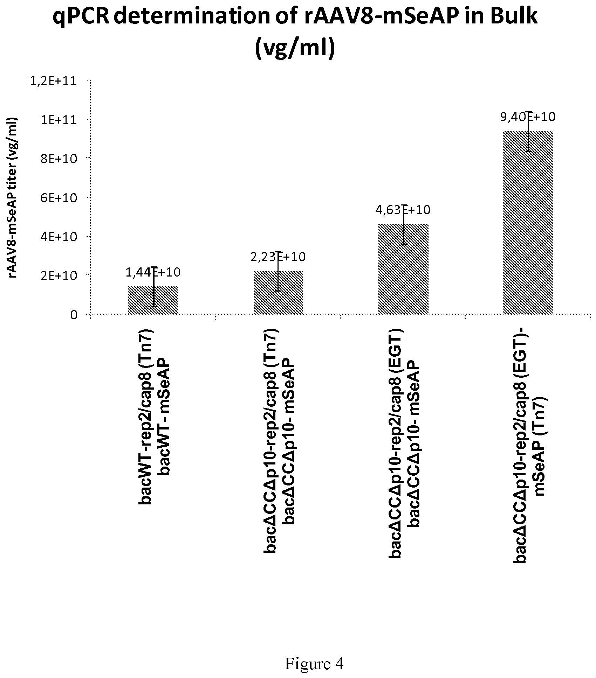

Des productions d'AAVr ont été réalisées en spinners dans des conditions identiques. Les titres d'AAVr ont ensuite été déterminés et sont indiqués figure 4. La production classique à 2 baculovirus (Smith et al., 2009) utilisant les baculovirus AcbacWT-rep2/cap8(Tn7) & AcbacWT-mSeAP(Tn7) nous permettent d'obtenir un titre de 1,44x10¹⁰ vg/ml d'AAVr. La production équivalente utilisant cette fois les baculovirus inactivés pour les gènes *chitinase, cathépsine* et *p10,* AcbacΔCCΔp10-rep2/cap8(Tn7) & AcbacΔCCΔp10-mSeAP(Tn7) permettent d'obtenir des titres de 2,23x10¹⁰ vg/ml. Ces résultats sont dans le même ordre de grandeur que ceux obtenus pour des productions effectuées avec le génome du bacmide non modifié. Afin de vérifier l'effet locus du déplacement de la cassette d'expression *rep2*/*cap8* du site Tn7 au locus *egt,* des productions ont été faites avec le baculovirus AcbacΔCCΔp10-rep2/cap8(EGT) et le baculovirus AcbacΔCCΔp10-mSeAP(Tn7). Le titre obtenu est de 4,63x10¹⁰ vg/ml d'AAVr, soit un doublement du titre obtenu en comparaison avec les productions où la cassette *rep2*/*cap8* est insérée au site Tn7 (p=0,005). Ce résultat montre un effet locus positif pour la production d'AAVr lorsque la cassette d'expression *rep2*/*cap8* est insérée au locus *egt* au lieu du site Tn7. Enfin, la production d'AAVr en utilisant un seul baculovirus,

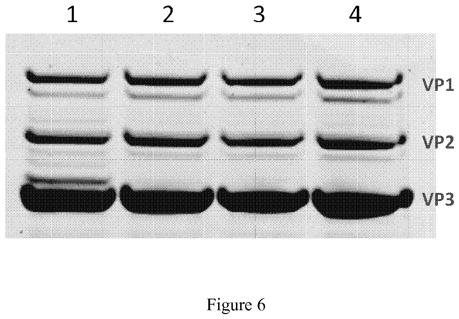

AcbacΔCCΔp10-rep2/cap8(EGT)-mSeAP(Tn7) a conduit à un 9,4x10¹⁰ vg/ml permettant ainsi une augmentation d'un facteur 6,5 (p=0,006) de la productivité en comparaison de la production d'AAVr réalisée avec deux baculovirus AcbacWT-rep2/cap8(Tn7) & AcbacWT-mSeAP(Tn7). Après purification des vecteurs produits, un facteur 4 de gain est toujours trouvé en faveur de la production d'AAVr réalisé avec un seul baculovirus, AcbacΔCCΔp10-rep2/cap8(EGT)-mSeAP(Tn7), en comparaison de la production d'AAVr réalisée avec deux baculovirus AcbacWT-rep2/cap8(Tn7) & AcbacWT-mSeAP(Tn7) (Figure 6).

Par ailleurs, l'analyse du profile protéique des vecteurs AAV recombinant purifiés montre la disparition de bandes de dégradations de la capside d'AAV recombinant associées à l'activité de la Cathépsine de baculovirus (Figure 6). Ces bandes sont visibles pour les vecteurs AAV produit avec le baculovirus sauvage (Figure 6, piste 1). Ces bandes de dégradation ne sont plus présentes dans les vecteurs AAV recombinant produits avec les baculovirus pour lesquels les gènes *chitinase, cathépsine* et *p10* sont éliminés du génome du baculovirus (Figure 6 pistes 2 ; 3 ; 4). Cette diminution de la dégradation des vecteurs AAV recombinant est associée à une augmentation de l'infectiosité *in vivo* pour ces vecteurs.

## Revendications

1. Génome baculoviral recombinant comprenant:
- une ou plusieurs cassettes d'expression des gènes *rep* et *cap* d'AAV nécessaires à la production d'un vecteur AAV, et
- un génome recombinant d'un vecteur AAV,
lesdites cassettes d'expression et ledit génome recombinant d'un vecteur AAV étant insérés dans un ou plusieurs locus choisi dans le groupe constitué des locus *egt, polyédrine, ctx, 39k, orf51, gp37, iap2* et *odv-e56, p10* et p94,ledit génome recombinant d'un vecteur AAV étant inséré dans un locus différent du(des) locus utilisés pour les gènes *rep* et *cap* d'AAV.

2. Génome baculoviral recombinant selon la revendication 1, lesdits gènes *rep* et *cap* étant contenus dans une cassette d'expression unique, notamment en orientation inverse, ladite cassette étant plus particulièrement insérée dans le locus *egt.*

3. Génome baculoviral recombinant selon la revendication 1 ou 2, la ou les cassettes d'expression des gènes *rep* et *cap* d'AAV comprenant un promoteur baculoviral très tardif, tel que le promoteur du gène polyédrine ou le promoteur du gène p10.

4. Génome baculoviral recombinant selon l'une quelconque des revendications 1 à 3, ledit génome recombinant d'un vecteur AAV étant inséré au niveau du locus *polyédrine*.

5. Génome baculoviral recombinant selon l'une quelconque des revendications 1 à 4, où:
- ledit génome baculoviral est dérivé d'AcMNPV; et/ou
- dans lequel les gènes *chitinase, cathépsine* et *p10* sont délétés.

6. Génome baculoviral recombinant selon la revendication 1 ou 5, où le génome recombinant AAV comprend un gène hétérologue codant une protéine, un ARN interférant ou un ARN antisens, en particulier thérapeutique.

7. Génome baculoviral recombinant selon l'une quelconque des revendications 1 à 6, ledit génome baculoviral recombinant étant un bacmide.

8. Baculovirus recombinant contenant un génome baculoviral recombinant selon l'une quelconque des revendications 1 à 6 à titre de génome.

9. Procédé pour produire un baculovirus recombinant, comprenant la culture d'une cellule procaryote contenant le bacmide recombinant défini dans la revendication 7 dans des conditions adaptées à la production d'un baculovirus.

10. Cellule eucaryote ou procaryote contenant le génome baculoviral recombinant selon l'une quelconque des revendications 1 à 7, ou cellule eucaryote infectée par le baculovirus recombinant de la revendication 8, ladite cellule étant en particulier une cellule d'insecte dérivée des lignées *Spodoptera frugiperda* ou *Trichoplusia ni,* par exemple les cellules Sf21, Sf9, TN 5B1-4 ou High Five.

11. Procédé pour produire un virus AAV recombinant, comprenant la mise en culture d'un baculovirus recombinant selon la revendication 8 avec une cellule, notamment une cellule d'insecte (par exemple une cellule Sf9, Sf21, TN 5B1-4 ou High Five), susceptible d'être infectée par ledit baculovirus, dans des conditions permettant l'infection de la cellule par le baculovirus et la production dudit virus AAV recombinant.

12. Procédé selon la revendication 11, comprenant la mise en culture d'un baculovirus unique comprenant dans son génome l'ensemble des éléments nécessaires à la production dudit AAV recombinant.

## Patentansprüche

1. Rekombinantes Baculovirus-Genom, umfassend:
- eine oder mehrere Expressionskassetten der *rep-* und *cap*-Gene von AAV, die für die Produktion eines AAV-Vektors erforderlich sind, und
- ein rekombinantes Genom eines AAV-Vektors,
wobei besagte Expressionskassetten und besagtes rekombinantes Genom eines AAV-Vektors in einen oder mehrere Loci inseriert sind, ausgewählt aus der Gruppe, bestehend aus den *egt, polyhedrin, ctx, 39k, orf51, gp37, iap2* und *odv-e56, p10* und *p94* Loci, wobei besagtes rekombinantes Genom eines AAV-Vektors in einen anderen Locus inseriert ist als der Locus oder die Loci, die für die *rep-* und *cap*-Gene von AAV verwendet werden.

2. Rekombinantes Baculovirus-Genom gemäß Anspruch 1, wobei besagte *rep-* und cap-Gene in einer einzigen Expressionskassette, besonders in inverser Orientierung, enthalten sind, wobei besagte Kassette insbesondere in den *egt* Locus inseriert ist.

3. Rekombinantes Baculovirus-Genom gemäß Anspruch 1 oder 2, wobei die Expressionskassette oder -kassetten der *rep-* und cap-Gene von AAV einen sehr späten Baculovirus-Promotor wie den Promotor des Polyhedrin-Gens oder den Promotor des p10-Gens umfasst oder umfassen.

4. Rekombinantes Baculovirus-Genom gemäß einem der Ansprüche 1 bis 3, wobei besagtes rekombinantes Genom eines AAV-Vektors auf Niveau des *polyhedrin* Locus inseriert ist.

5. Rekombinantes Baculovirus-Genom gemäß einem der Ansprüche 1 bis 4, wobei:
- besagtes Baculovirus-Genom von AcMNPV abstammt; und/oder
- in dem die *chitinase-, cathepsin*- und *p10*-Gene deletiert sind.

6. Rekombinantes Baculovirus-Genom gemäß Anspruch 1 oder 5, wobei das AAV rekombinante Genom ein heterologes Gen umfasst, das insbesondere therapeutisch ist und ein Protein, eine interferierende RNA oder eine antisense-RNA codiert.

7. Rekombinantes Baculovirus-Genom gemäß einem der Ansprüche 1 bis 6, wobei besagtes rekombinantes Baculovirus-Genom ein Bacmid ist.

8. Rekombinantes Baculovirus, enthaltend ein rekombinantes Baculovirus-Genom gemäß einem der Ansprüche 1 bis 6 als Genom.

9. Verfahren zur Produktion eines rekombinanten Baculovirus, umfassend die Kultivierung einer prokaryotischen Zelle, enthaltend das in Anspruch 7 definierte rekombinante Bacmid, unter Bedingungen, die für die Produktion eines Baculovirus geeignet sind.

10. Eukaryotische oder prokaryotische Zelle, enthaltend das rekombinante Baculovirus-Genom gemäß einem der Ansprüche 1 bis 7, oder eukaryotische Zelle, infiziert mit dem rekombinanten Baculovirus nach Anspruch 8, wobei besagte Zelle insbesondere eine Insektenzelle ist, die von den Linien *Spodoptera frugiperda* oder *Trichoplusia ni* abstammt, zum Beispiel die Sf21-, Sf9-, TN 5B1-4- oder High Five-Zellen.

11. Verfahren zur Produktion eines rekombinanten AAV-Virus, umfassend die Kultivierung eines rekombinanten Baculovirus gemäß Anspruch 8 mit einer Zelle, besonders einer Insektenzelle (zum Beispiel einer Sf21-, Sf9-, TN 5B1-4- oder High Five-Zelle), die mit besagtem Baculovirus infiziert werden kann, unter Bedingungen, die die Infektion der Zelle mit dem Baculovirus und die Produktion besagten rekombinanten AAV-Virus erlauben.

12. Verfahren gemäß Anspruch 11, umfassend die Kultivierung eines einzigen Baculovirus, umfassend in seinem Genom die gesamten Elemente, die für die Produktion besagten rekombinanten AAV erforderlich sind.

## Claims

1. A recombinant baculovirus genome comprising:
- one or more expression cassettes for the AAV *rep* and *cap* genes required for the production of an AAV vector, and
- a recombinant genome of an AAV vector,
said expression cassettes and said recombinant genome of an AAV vector being inserted into one or more loci selected from the group consisting of the *egt, polyhedrin, ctx, 39k, orf51, gp37, iap2* and *odv-e56*, *p10* and *p94* loci, said recombinant genome of an AAV vector being inserted into a locus different than the locus or loci used for the AAV *rep* and *cap* genes.

2. The recombinant baculovirus genome according to claim 1, said *rep* and *cap* genes being contained in a single expression cassette, in particular in reverse orientation, said cassette being more particularly inserted into the *egt* locus.

3. The recombinant baculovirus genome according to claim 1 or 2, the expression cassette(s) for the AAV *rep* and *cap* genes comprising a very late baculovirus promoter, such as the polyhedrin gene promoter or the p10 gene promoter.

4. The recombinant baculovirus genome according to any one of claims 1 to 3, said recombinant genome of an AAV vector being inserted at the level of the *polyhedrin* locus.

5. The recombinant baculovirus genome according to any one of claims 1 to 4, wherein:
- said baculovirus genome is derived from AcMNPV; and/or
- wherein the *chitinase, cathepsin* and *p10* genes are deleted.

6. The recombinant baculovirus genome according to claim 1 or 5, wherein the AAV recombinant genome comprises a heterologous gene encoding a protein, an interfering RNA or an antisense RNA, which is in particular therapeutic.

7. The recombinant baculovirus genome according to any one of claims 1 to 6, said recombinant baculovirus genome being a bacmid.

8. A recombinant baculovirus containing as genome a recombinant baculovirus genome according to any one of claims 1 to 6.

9. A process for producing a recombinant baculovirus, comprising culturing a prokaryotic cell containing the recombinant bacmid defined in claim 7 under conditions suitable for the production of a baculovirus.

10. A eukaryotic or prokaryotic cell containing the recombinant baculovirus genome according to any one of claims 1 to 7, or a eukaryotic cell infected with the recombinant baculovirus of claim 8, said cell being in particular an insect cell derived from the *Spodoptera frugiperda* or *Trichoplusia ni* lines, for example the Sf21, Sf9, TN 5B1-4 or High Five cells.

11. A process for producing a recombinant AAV virus, comprising culturing a recombinant baculovirus according to claim 8 with a cell, in particular an insect cell (for example an Sf9, Sf21, TN 5B1-4 or High Five cell), capable of being infected with said baculovirus, under conditions which allow the infection of the cell with the baculovirus and the production of said recombinant AAV virus.

12. The process according to claim 11, comprising culturing a single baculovirus comprising in its genome all of the elements required for the production of said recombinant AAV.


### SEQUENCE LISTING

<110> Genethon
<120> SYSTEME BACULOVIRAL POUR L'EXPRESSION D'UN VECTEUR DE THERAPIE GENIQUE
<130> B1215PC00
<160> 15
<170> PatentIn version 3.5
<210> 1
   <211> 120
   <212> ADN
   <213> Artificielle
<220>
   <223> amorce
<400> 1
   ttacggtcgt caagcccaaa ctgtttgcgt attcaactaa aacttattgc ggtaatatca       60
   ctaccgttcg tatagcatac attatacgaa gttataatag gaacttcatt taaatggcgc      120
<210> 2
   <211> 111
   <212> ADN
   <213> Artificielle
<220>
   <223> amorce
<400> 2
   tcccggcttc caaggcctcg tcgctcgatt gtagtccgcc ttgcgtaata aacgccgcca       60
   tttttttatg acgcagcacg gcagacatga taagatacat tgatgagttt g               111
<210> 3
   <211> 19
   <212> ADN
   <213> Artificielle
<220>
   <223> amorce
<400> 3
   atgactattc tctgctggc 19
<210> 4
   <211> 18
   <212> ADN
   <213> Artificielle
<220>
   <223> amorce
<400> 4
   attggccgtg tttcctac 18
<210> 5
   <211> 22
   <212> ADN
   <213> Artificielle
<220>
   <223> amorce
<400> 5
   ccagtcacga cgttgtaaaa cg 22
<210> 6
   <211> 23
   <212> ADN
   <213> Artificielle
<220>
   <223> amorce
<400> 6
   agcggataac aatttcacac agg 23
<210> 7
   <211> 20
   <212> ADN
   <213> Artificielle
<220>
   <223> amorce
<400> 7
   agccacctac tcccaacatc 20
<210> 8
   <211> 114
   <212> ADN
   <213> Artificielle
<220>
   <223> amorce
<400> 8
   ccgctgttga aacaatattt tataataccc tgtttatagt taacaatgtc ggcagcgtct 60
   atggccatag gaatagggcc taccgttcgt ataatgtatg ctatacgaag ttat 114
<210> 9
   <211> 114
   <212> ADN
   <213> Artificielle
<220>
   <223> amorce
<400> 9
   ccgctgttga aacaatattt tataataccc tgtttatagt taacaatgtc ggcagcgtct 60
   atggccatag gaatagggcc taccgttcgt ataatgtatg ctatacgaag ttat 114
<210> 10 <211> 24
   <212> ADN
   <213> Artificielle
<220>
   <223> amorce
<400> 10
   cgcggccgta catggcgacg ccca 24
<210> 11
   <211> 25
   <212> ADN
   <213> Artificielle
<220>
   <223> amorce
<400> 11
   gtttttaaag gtccaatatg gaatg 25
<210> 12
   <211> 84
   <212> ADN
   <213> Artificielle
<220>
   <223> amorce
<400> 12
   ttgtatatta attaaaatac tatactgtaa attacatttt atttacaatc taccgttcgt 60
   atagcataca ttatacgaag ttat 84
<210> 13
   <211> 84
   <212> ADN
   <213> Artificielle
<220>
   <223> amorce
<400> 13
   gaatcgtacg aatattataa aacaattgat ttgttatttt aaaaacgatt taccgttcgt 60
   ataatgtatg ctatacgaag ttat 84
<210> 14
   <211> 25
   <212> ADN
   <213> Artificielle
<220>
   <223> amorce
<400> 14
   ccgggacctt taattcaacc caaca 25
<210> 15
   <211> 25
   <212> ADN
   <213> Artificielle
<220>
   <223> amorce
<400> 15
   cagcatttgt tatacacaca gaact 25